(19) Europäisches Patentamt
European Patent Office
Office européen des brevets

(11) **EP 2 758 520 B1**

(12) **FASCICULE DE BREVET EUROPEEN**

(45) Date de publication et mention de la délivrance du brevet:
**27.12.2017 Bulletin 2017/52**

(51) Int Cl.:
*C12N 5/00* *(2006.01)* *B29K 67/00* *(2006.01)*
*B29C 39/02* *(2006.01)* *B29C 39/42* *(2006.01)*
*B29K 105/00* *(2006.01)* *G03F 7/00* *(2006.01)*
*C12N 5/077* *(2010.01)*

(21) Numéro de dépôt: **12773047.1**

(22) Date de dépôt: **17.09.2012**

(86) Numéro de dépôt international:
**PCT/FR2012/052073**

(87) Numéro de publication internationale:
**WO 2013/041800 (28.03.2013 Gazette 2013/13)**

(54) **DISPOSITIF DE GUIDAGE DE LA MIGRATION CELLULAIRE ET METHODE DE GUIDAGE METTANT EN OEUVRE UN TEL DISPOSITIF**

VORRICHTUNG ZUR FÜHRUNG EINER ZELLMIGRATION SOWIE FÜHRUNGSVERFAHREN MIT EINER SOLCHEN VORRICHTUNG

DEVICE FOR GUIDING CELL MIGRATION AND GUIDING METHOD IMPLEMENTING SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.09.2011 FR 1158316**

(43) Date de publication de la demande:
**30.07.2014 Bulletin 2014/31**

(73) Titulaires:
• **Institut Curie**
**75005 Paris (FR)**
• **Centre National de la Recherche Scientifique (C.N.R.S.)**
**75794 Paris Cedex 16 (FR)**
• **URGO RECHERCHE INNOVATION ET DEVELOPPEMENT**
**21300 Chenôve (FR)**

(72) Inventeurs:
• **LE BERRE, Maël**
**F-75020 Paris (FR)**
• **PIEL, Matthieu**
**F-75012 Paris (FR)**
• **CHEN, Yong**
**F-75020 Paris (FR)**
• **LIU, Yanjun**
**Shanghai 200032 (CN)**

(74) Mandataire: **Cabinet Plasseraud**
**66, rue de la Chaussée d'Antin**
**75440 Paris Cedex 09 (FR)**

(56) Documents cités:
**US-A1- 2009 248 145**

• **GOHER MAHMUD ET AL: "Directing cell motions on micropatterned ratchets", NATURE PHYSICS, vol. 5, no. 8, 1 août 2009 (2009-08-01), pages 606-612, XP055034675, ISSN: 1745-2473, DOI: 10.1038/nphys1306 cité dans la demande**
• **HWA SENG KHOO ET AL: "Engineering the 3D architecture and hydrophobicity of methyltrichlorosilane nanostructures", NANOTECHNOLOGY, IOP, BRISTOL, GB, vol. 19, no. 34, 27 août 2008 (2008-08-27), page 345603, XP020144505, ISSN: 0957-4484, DOI: 10.1088/0957-4484/19/34/345603**
• **SE YON HWANG ET AL: "Adhesion Assays of Endothelial Cells on Nanopatterned Surfaces within a Microfluidic Channel", ANALYTICAL CHEMISTRY, vol. 82, no. 7, 1 avril 2010 (2010-04-01), pages 3016-3022, XP055034676, ISSN: 0003-2700, DOI: 10.1021/ac100107z**

## Description

La présente invention a pour objet un dispositif de guidage de la migration cellulaire comprenant un substrat présentant une surface texturée ayant une structure tridimensionnelle anisotrope destinée à être mise en contact avec des cellules.

La migration des cellules est essentielle pour de nombreux processus physiologiques comme l'organogénèse ou la cicatrisation des plaies. Dans leur environnement naturel, la direction et la vitesse de migration des cellules sont guidées par de nombreux signaux qui peuvent être chimiques (chimiokines) ou physiques (microenvironnement).

In vitro, ces phénomènes peuvent être reproduits ou détournés pour imposer une direction de migration aux cellules en utilisant, par exemple, des chemoattractants, des champs électriques, ou encore en modulant l'environnement mécanique de la cellule.

Le document EP-A-1199354 décrit, par exemple, la formation d'un motif de cellule sur une surface par un contrôle chimique de la migration des cellules. En effet, dans le document EP-A-1199354, la surface est traitée de manière à présenter un pré-motif constitué de composés favorisant le développement des cellules, et d'autres composés ne favorisant pas le développement des cellules. La culture des cellules est ensuite initiée sur ce pré-motif. Cependant, l'efficacité du contrôle de la migration des cellules par ce type de système dépend principalement du choix des composés chimiques favorisant ou empêchant le développement cellulaire en fonction de la nature des cellules cultivées.

Le document US 2007/0009572 décrit, quant à lui, une méthode de préparation d'un film biodégradable micro- ou nano-texturé comprenant des canaux dont la largeur peut varier de 10 à 160 $\mu$m, sur lequel sont déposées des cellules musculaires. Les essais réalisés montrent que les cellules musculaires s'alignent les unes par rapport aux autres le long des canaux, et que leur morphologie se modifie pour prendre une forme allongée. Cette méthode n'a pas pour finalité de faire migrer les cellules dans une direction préférée, mais juste de favoriser leur alignement les unes par rapport aux autres pour obtenir un empilement cellulaire régulier.

Le document US 2009/02481445 décrit également une méthode pour guider l'orientation des cellules suivant une structure tridimensionnelle en utilisant une surface comprenant un micro-canal ou une série de micro-canaux parallèles les uns aux autres, dont la largeur est supérieure à celle des cellules afin que les cellules puissent s'y introduire et dont la section est arbitraire. Comme pour le document précédent, cette méthode n'a pas pour finalité de faire migrer les cellules dans une direction préférée, mais juste de favoriser leur alignement les unes par rapport aux autres.

Mahmud et al. (Nature Physics 2009, 4, pp. 606) proposent des motifs adhésifs en forme de cliquets (« ratchet ») pour guider la migration cellulaire. L'effet observé est basé sur un contraste d'adhésion entre les parties adhésives des canaux et les parties non-adhésives d'un substrat de sorte que, lorsque la qualité du contraste entre les parties adhésives et non-adhésives se dégrade avec le temps, le guidage de la migration cellulaire n'est plus observé. En outre, l'adhésion sur les canaux, linéaires ou présentant une forme de cliquets (« ratchet »), ne permet de maintenir les cellules que sur ces motifs adhésifs, c'est-à-dire sur une seule dimension dans l'espace, et ne permet pas, par exemple, l'organisation d'un tissu sur une surface en deux dimensions. Enfin, les motifs décrits dans Mahmud *et al.* sont toujours des projections perpendiculaires au plan constitué par la surface véhiculant les cellules.

Ces méthodes permettant de détourner les phénomènes naturels de migration cellulaire peuvent également trouver des applications in vivo.

Le document US2009/0093879 propose notamment un implant présentant en surface des motifs tridimensionnels micro- ou nanométriques. Ces motifs permettent notamment de contrôler l'adhésion de micro-organismes ou de fibroblastes à la surface de l'implant lorsque celui-ci est mis en place chez un être vivant, améliorant ainsi la cicatrisation des blessures.

Ce document US2009/0093879 suggère que les micro- ou nanostructures de surface peuvent guider les cellules à l'origine de la cicatrisation, leur permettant ainsi de s'organiser de manière ordonnée à la surface de l'implant.

Or un tel contrôle de la migration cellulaire selon une direction donnée pourrait également présenter des applications dans le domaine médical autres que l'organisation forcée des cellules autour d'un implant, telles que la migration dirigée des cellules à la surface d'une plaie ou la réalisation d'organes artificiels en ingénierie tissulaire.

Il existe donc un besoin pour de nouveaux dispositifs permettant de guider les cellules dans leur migration selon une direction choisie, dont l'efficacité ne dépend pas du type de cellule motile considérée, simples à mettre en oeuvre, peu invasifs pour les tissus et robuste dans le temps.

Par guider la migration cellulaire on entend, au sens de la présente demande, que l'on impose aux cellules de migrer préférentiellement dans une direction plutôt que dans toutes les autres. En d'autres termes, le guidage de la migration brise la symétrie de migration suivant la direction considérée. Le « guidage » de la migration diffère de « l'orientation » de la migration cellulaire où les cellules migrent préférentiellement dans deux directions opposées sans que l'une de ces directions ne soit favorisée par rapport à l'autre.

Un objet de la présente invention est donc de proposer un dispositif de guidage de la migration cellulaire comprenant un substrat présentant une surface texturée destinée à être mise en contact avec des cellules, ladite surface texturée ayant une structure tridimensionnelle anisotrope constituée d'un réseau de projections inclinées par rapport à la normale au plan constitué par ladite surface texturée, dans la direction conférée par ladite structure anisotrope.

Contrairement aux documents de l'art antérieur décrivant des canaux ou des micro-canaux contraignant l'alignement des cellules, la présente invention permet le guidage des cellules selon une direction d'anisotropie formant ainsi un réseau dans le plan compatible avec l'organisation d'un tissus selon une surface donnée.

L'invention a également pour objet, selon un second aspect, une méthode de guidage de la migration des cellules comprenant la mise en contact des cellules avec un substrat présentant une surface texturée ayant une structure tridimensionnelle anisotrope, ladite structure étant constituée d'un réseau de projections inclinées telles que décrites précédemment.

Enfin, l'invention a pour objet, selon un autre aspect, un kit de guidage de la migration cellulaire, comprenant un substrat présentant une surface texturée telle que décrit précédemment, ayant une structure tridimensionnelle anisotrope constituée d'un réseau de projections inclinées par rapport à la normale au plan constitué par ladite surface texturée, dans la direction conférée par ladite structure anisotrope, et une surface support sur laquelle des cellules sont destinée à être véhiculées.

Le dispositif ou la méthode selon l'invention peut notamment trouver des applications dans les domaines de la dermatologie, de l'implantologie ou de l'ingénierie tissulaire.

Par « structure anisotrope » ou « structure à géométrie anisotrope », on entend, au sens de la présente demande, une structure dont la géométrie possède une direction d'anisotropie déterminée selon un axe donné.

La direction d'anisotropie de la structure anisotrope est notamment, dans le cadre de la présente invention, la direction de la migration des cellules.

Le dispositif selon l'invention met en oeuvre un substrat présentant une surface texturée dont la structure tridimensionnelle est constituée d'un réseau de projections inclinées.

Description des figures

La figure 1 illustre une surface texturée comprenant un réseau de projections inclinées de forme cylindrique.
La figure 2 illustre le confinement des cellules entre la surface texturée et la surface support.
La figure 3 illustre différentes utilisations du dispositif selon l'invention :

Figure 3A) : des structures adhésives simples pour guider les cellules dans une direction.
Figure 3B) : le confinement des cellules entre la surface texturée et la surface support dans un espace d'environ 5 μm.
Figure 3C) : l'application de la surface texturée sur un substrat mou type gel.
Figure 3D) : l'application de la surface texturée sur un tissu vivant.

La figure 4 illustre les différentes étapes du procédé de préparation de la surface texturée selon l'invention:

La figure 4A) illustre une plaque de verre sur laquelle est déposée une couche de chrome recouverte par une couche de résine photosensible.
Les figures 4 (B) et (C) illustrent la formation des motifs sur la couche photosensible par un procédé photolithographique et son transfert dans la couche de chrome par gravure.
La figure 4 (D) illustre l'application d'une couche de résine sur les motifs formés.
La figure 4 (E) illustre l'irradiation UV de la couche de résine.
La figure 4 (F) illustre les projections inclinées formées suite à l'irradiation UV.
La figure 4 (G) illustre le dépôt consécutif d'une couche de silicone PDMS/
La figure 4 (H) illustre le moule de PDMS en négatif ainsi formé.
La figure 4 (I) illustre l'application d'une couche du matériau constituant la surface texturée selon l'invention sur le moule de PDMS en négatif.
La figure 4 (J) illustre la surface tridimensionnelle ainsi formée.

La figure 5 présente une photographie par microscopie optique d'une surface texturée selon l'invention.
La figure 6 représente un histogramme de la direction moyenne de migration des cellules pendant 24h.

Substrat

Dans le cadre de la présente demande, l'effet technique de guidage de la migration cellulaire est obtenu au moyen de la surface texturée particulière telle que décrit précédemment, ayant une structure tridimensionnelle anisotrope constituée d'un réseau de projections inclinées par rapport à la normale au plan constitué par ladite surface texturée, dans la direction conférée par ladite structure anisotrope, lorsque cette surface texturée est mise en contact avec des cellules.

Le substrat présentant une surface texturée, notamment micro- ou nano-texturée, du dispositif selon l'invention peut être adhésif ou non adhésif.

Le choix d'un substrat adhésif peut notamment permettre d'améliorer encore l'effet technique de guidage de la migration cellulaire déjà obtenu grâce au dispositif mettant en oeuvre la surface spécifique selon l'invention.

Les matériaux adhésifs susceptibles de convenir pour le substrat selon l'invention peuvent notamment être hydrophiles ou hydrophobes, en étant, le cas échéant, traités avec un promoteur d'adhésion cellulaire, et notamment choisis parmi :

- les plastiques biocompatibles (par exemple le polystyrène (PS), couramment utilisé en culture cellulaire, des polymères siliconés comme le polydiméthylesiloxane (PDMS) notamment utilisés dans les laboratoires sur puce, des gels de copolymères blocks comme le styrène-éthylène/butylène-styrène (SEBS) utilisés pour la fabrication de pansements ou les polyacides lactiques et glycoliques (PLGA, PLA : hydrophile) qui sont biodégradables et peuvent être utilisés pour des implants ou comme support de tissus artificiels). Certains de ces plastiques peuvent avantageusement être activés par plasma oxygène pour augmenter leur hydrophilie et promouvoir l'adhésion cellulaire.

- les céramiques, généralement hydrophiles, telles que les oxydes métalliques ou les nitrures, comme par exemple le verre ($SiO_2$), le nitrure de silicium ($Si_3N_4$), le dioxyde de titane ($TiO_2$) ou d'autres. Ces matériaux sont utilisés en culture cellulaire, dans les laboratoires sur puce ou en implantologie. Ces matériaux peuvent avantageusement être activés par plasma oxygène pour augmenter leur hydrophilie et promouvoir l'adhésion cellulaire.

- Les métaux inertes comme l'or, le platine, le palladium ou les métaux dont la surface oxydée ou nitrurée est stable comme le chrome ou le titane qui sont utilisés pour les implants. Avantageusement, les métaux peuvent être traités avec des molécules de la famille des thiols pour augmenter ou diminuer leur capacité d'adhésion cellulaire.

Il est possible de promouvoir l'adhésion cellulaire, en traitant chimiquement le matériau support. On peut alors utiliser:

- des polymères chargés (polyélectrolytes) qui s'adsorbent fortement par interaction électrostatique sur les surfaces oxydées (naturellement comme pour les oxydes ou artificiellement en activant les surfaces à l'aide d'un plasma oxygène). Par exemple la Poly-L-lysine (PLL) ou la Polyhornitine (PORN) ; ou
- des protéines d'adhésion cellulaire (Intégrines) ou de la matrice extracellulaire (Fibronectine, laminine, collagène) ou des peptides mimant ces protéines, comme le motif RGD (Arginyl glycyl aspartic acid).

Il est également possible, dans le cadre de la présente invention, de moduler l'adhésion du substrat pour optimiser la motilité des cellules. En effet, le niveau d'adhésion des cellules sur un substrat peut être modulé en traitant ce substrat adhésif avec un mélange ratiométrique de molécules adhésives et de molécules non-adhésives. Par exemple, un mélange de pLL-PEG et de pLL-PEG-RGD ou un mélange de pLL-PEG et de Fibronectine peuvent être utilisés.

Selon un mode préféré de réalisation, le substrat présentant une surface texturée est un substrat non adhésif, c'est-à-dire sur lequel les cellules ne peuvent pas adhérer, de manière à pouvoir être retiré sans détériorer les cellules. Ces substrats non adhésifs sont aussi appelés substrats anti-encrassement (ou « anti-fouling » selon la terminologie anglosaxone).

Le caractère non adhésif du substrat correspond à une faible capacité d'adsorption protéique dudit substrat ainsi qu'une faible capacité d'adhésion cellulaire, ce qui permet généralement de limiter les réactions inflammatoires.

Les matériaux non adhésifs pouvant convenir au substrat présentant une surface texturée selon l'invention peuvent notamment être des matériaux superhydrophobes - comme c'est le cas des polymères fluorés (par exemple le polytétrafluoroéthylène (PTFE)) - ou des gels comme le polyacrylamide (PAM) ou le polyéthylèneglycol-diacrylate (PEGDA).

Alternativement, le substrat non adhésif peut être constitué d'un matériau rendu non adhésif par traitement chimique.

Les traitements chimiques permettant de rendre le substrat non adhésif peuvent notamment être le greffage sur le substrat d'une couche de gel mono-moléculaire, par exemple de type polyéthylène glycol (PEG), par exemple un PEG silanisé sur les oxydes ou thiolé sur les métaux ou conjugué avec un polyélectrolyte pour lui conférer la capacité de s'adsorber durablement par interaction électrostatique sur le substrat, comme c'est le cas pour le greffage de Polylysine-PEG (PLL-PEG).

De préférence, le matériau non adhésif est un polymère fluoré ou un matériau rendu non adhésif par traitement chimique tel que le greffage de molécules, par exemple de polyéthylène glycol (PEG).

Surface texturée

La surface texturée du dispositif selon l'invention présente une structure tridimensionnelle anisotrope constituée d'un réseau de projections inclinées.

Par « réseau » de projections inclinées, on entend, au sens de la présente demande que les projections inclinées sont disposées de manière périodique sur la surface texturée de manière à produire un motif de répétition régulier.

La direction d'inclinaison des projections permet notamment d'imposer une direction de moindre friction, déterminant ainsi la direction de déplacement préférentielle des cellules. Selon un mode particulier de réalisation, pour améliorer la friction, le réseau de projections présente une période, définie comme la distance entre deux projections, inférieure à la taille des cellules, de manière à ce que la cellule soit toujours en contact avec au moins deux projections, de préférence inférieure à 20 μm, de manière à éviter que les cellules ne circulent de manière aléatoire entre les projections.

Dans le cadre de l'invention, la surface texturée présente une dimension supérieure à la taille des cellules.

De préférence, la période du réseau de projections est comprise entre 0,1 et 15 μm, de préférence encore comprise entre 5 et 10 μm et plus préférentiellement de l'ordre de 5 μm.

Dans le cadre de l'invention, les projections du réseau de la structure tridimensionnelle anisotrope constituant la surface texturée sont inclinées par rapport à la normale au plan constitué par ladite surface texturée dans la direction conférée par ladite structure anisotrope.

En particulier, ces projections présentent de préférence un angle d'inclinaison inférieur ou égal à 45° par rapport à la normale au plan constitué par la surface texturée, et de préférence encore entre 10° et 45°.

Les projections sont de préférence de taille nanométrique, c'est-à-dire que leur hauteur, mesurée à partir du substrat de la surface texturée, est de l'ordre du nanomètre, en particulier entre 100nm et 20μm, de manière à limiter l'indentation et éviter le perçage de la cellule sous la pression des projections.

En particulier, les projections présentent de préférence un rapport d'aspect, correspondant au rapport de la hauteur sur le diamètre des projections, compris entre 0,5 et 20, de préférence entre 2 et 10.

Avantageusement, ce rapport d'aspect peut être utilisé pour ajuster la rigidité du substrat perçu par les cellules en utilisant le fait que plus les piliers présentent un rapport d'aspect élevé, moins les projections sont rigides, et donc plus elles vont se déformer facilement sous l'effet des forces appliquées par les cellules.

Par exemple, des micro- ou nano-piliers moulés dans un substrat de type PDMS standard, de rigidité 2 MPa permettront d'obtenir des rigidités allant de 1,7 kPa pour un rapport d'aspect de 6,25 à 61 kPa pour un rapport d'aspect de 1,9 (Thèse de S. Ghassemi, Columbia University, 2011, pp 38, (http://academiccommons.columbia.edu/catalog/ac:131397)).

Or, la rigidité des projections permet d'induire un certain phénotype, une certaine différentiation ou la prolifération des cellules en contact avec le substrat, tout en conservant la propriété de guidage des cellules recherchée selon la présente demande. En effet, il a été montré que certaines cellules, lorsqu'elles évoluent sur des substrats plus rigides, ont tendance à se différencier en cellules osseuses, alors que ces mêmes cellules, lorsqu'elles évoluent dans des substrats plus souples, ont tendance à se différencier en cellules neuronales.

Par conséquent, il peut être intéressant, dans le cadre de la présente demande, d'ajuster le rapport d'aspect des projections de la surface texturée selon l'invention (notamment lorsque les cellules sont véhiculées sur cette surface) de manière à modifier le phénotype, la différentiation ou la prolifération desdites cellules.

Les projections inclinées peuvent se présenter sous toute forme géométrique, et notamment sous la forme de cylindres, de cônes, de pyramides, de lamelles, ou d'écailles de forme sensiblement triangulaire, semi-elliptique ou semi-circulaire.

Lorsque les projections se présentent sous la forme de lamelles, leur section peut être rectangulaire ou de la forme d'un parallélogramme, la section étant définie par un plan normal à l'axe de ladite lamelle.

Selon un mode préféré de réalisation, les projections inclinées se présentent sous la forme de cylindres pouvant notamment présenter une section sphérique ou ovoïdale, la section étant définie par un plan normal à l'axe dudit cylindre.

Au sens de la présente demande, la section des projections, quelque soit sa forme, s'inscrit dans un cercle de diamètre déterminé, défini comme étant le diamètre desdites projections.

Lorsque le diamètre des projections n'est pas constant (par exemple lorsque les projections sont coniques), le diamètre est mesuré pour une section située à mi-hauteur des projections.

Les projections inclinées présentent de préférence un diamètre compris entre 10 nm et 10 μm, de préférence entre 0,5 et 3 μm.

Selon un mode particulier de réalisation, la surface occupée par les projections inclinées, disposées de manière périodique sur la surface texturée, représente de 5 à 50% de la surface texturée, et de préférence de l'ordre de 5% de la surface texturée.

De telles structures anisotropes constituées d'un réseau de projections inclinées peuvent, par exemple, être obtenues par photolithographie ou par nanolithographie (« nanoimprint » en terminologie anglo-saxonne), optionnellement suivie par une étape de gravure sèche anisotrope telle qu'une gravure ionique réactive (GIR ou RIE pour « Reactive-Ion Etching ») incluant les systèmes de torche à plasma (« Inductively Coupled Plasma », ICP) ou DRIE (« Deep Reactive-Ion Etching ») en inclinant l'échantillon lors de la photolithographie ou lors de la gravure pour obtenir l'inclinaison des structures.

Fabrication de la surface texturée

La surface texturée du dispositif selon l'invention, présentant une structure tridimensionnelle anisotrope constituée d'un réseau de projections inclinées, peut être préparée par toute méthode connue de l'homme du métier.

Il est cependant du mérite de la demanderesse d'avoir mis au point une nouvelle méthode de préparation de ce type de surfaces.

Les projections inclinées peuvent en effet être réalisées par coulage d'un polymère siliconé (PDMS) ou par moulage d'un copolymère poly(acide lactic-co- glycolique) (PLGA) sur un moule en silicone (PDMS).

Dans le cadre de l'invention, les projections inclinées, lorsqu'elles sont utilisées pour le guidage des cellules par confinement, peuvent de préférence être réalisées en un polymère siliconé (PDMS), dont la surface est rendue non adhésive.

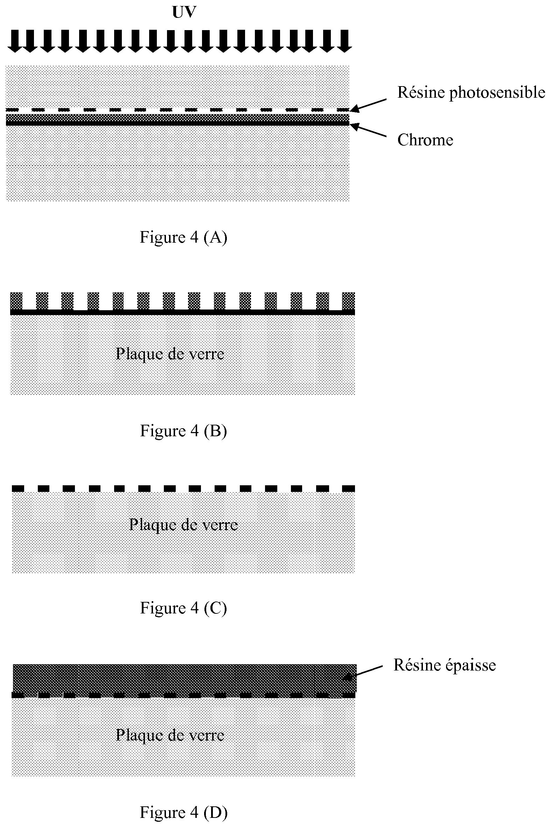

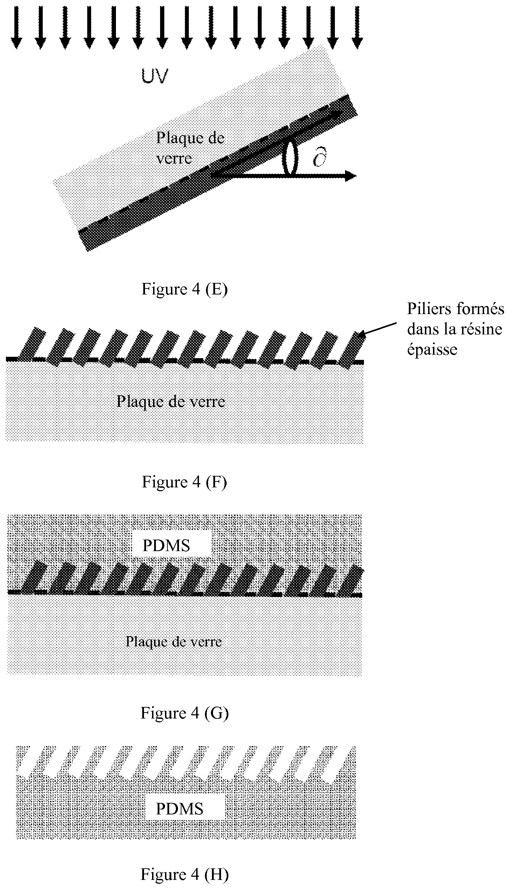

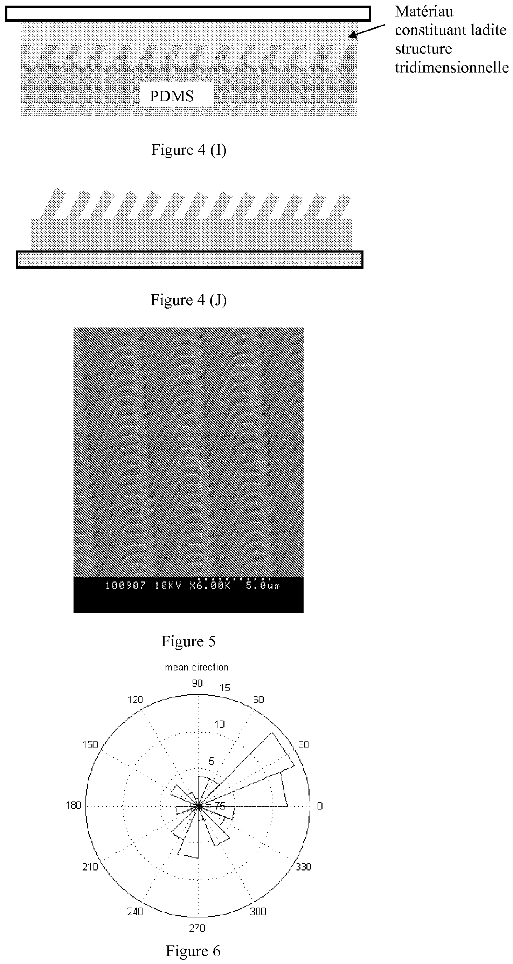

Selon un autre aspect particulier de l'invention, les projections inclinées, lorsqu'elles sont utilisées pour le guidage des cellules sans confinement, peuvent de préférence être réalisées par moulage d'un copolymère poly(acide lactic-co- glycolique) (PLGA) sur un moule en silicone (PDMS) comme illustré à la figure 4.

Ainsi, l'invention a pour objet, selon un aspect particulier, un procédé de préparation d'une surface texturée ayant une structure tridimensionnelle anisotrope constituée d'un réseau de projections inclinées par rapport à la normale au plan constitué par ladite surface texturée, dans la direction conférée par ladite structure anisotrope, comprenant :

> i. la préparation de moules en silicone constituant des copies en négatif de ladite structure tridimensionnelle, notamment par lithographie optique auto-alignée,
> ii. le moulage du matériau constituant ladite structure tridimensionnelle,
> iii. le décollement du moule en silicone.

En particulier, des moules en silicone (PDMS) peuvent être préparés par lithographie optique auto-alignée, comprenant notamment les étapes suivantes :

> i. le dépôt, d'une couche de résine photosensible sur un masque optique,
> ii. l'irradiation de ladite couche de résine à travers le masque optique par une source lumineuse, avec un angle incident (correspondant à l'angle formé par le rayon incident et la normale au plan constitué par le masque), ledit angle incident conditionnant l'angle d'inclinaison des piliers de la structure tridimensionnelle anisotrope,
> iii. optionnellement, le recouvrement de ladite surface tridimensionnelle de résine par un matériau antiadhésif,
> iv. le coulage d'une couche de PDMS sur ladite surface tridimensionnelle de résine de manière à obtenir lesdits moules en silicone (PDMS),
> v. après polymérisation, décollement du moule de PDMS ainsi obtenu (Figure 4 (H))

Le masque optique utilisé à l'étape i. peut être un substrat transparent usuellement utilisé pour les masques optiques, comme par exemple une plaque de verre, sur lequel sont réalisés des motifs constituant le masque en un matériau métallique absorbant la lumière, par exemple du chrome. Ces masques sont classiques pour la photolithographie et disponibles dans le commerce ou peuvent être réalisés par des méthodes connues de l'homme du métier.

L'homme du métier peut, par exemple, réaliser un masque optique en déposant une couche métallique absorbant la lumière (notamment du chrome) sur un substrat transparent tel qu'une plaque de verre et en réalisant des motifs (par exemple une matrice de trous) sur ladite couche métallique, par exemple par gravure avec une solution gravant le chrome comme le ChromeEtch, commercialisé par MicroChem.

Pour cela, on réalise des motifs par balayage d'un faisceau laser focalisé sur une couche de résine photosensible déposée sur le substrat transparent (plaque de verre) préalablement recouvert préalablement d'une couche de chrome. Après révélation de la résine, on transfère ces motifs sur la couche de chrome en trempant le substrat de verre dans un bain de ChromeEtch. Pour finir, les motifs de résine ayant servi de masque sont éliminés à l'aide d'un solvant adapté. Un masque vierge de type PR-AZ1518 Cr (plaque de verre couverte d'une couche fine de chrome et d'une couche de résine), commercialisé par Micro Chem, peut, par exemple, être utilisé pour la réalisation de masques optiques.

À l'étape i, l'épaisseur de la couche de résine appliquée est notamment choisie selon la hauteur des piliers souhaitée. La résine est, par exemple, une résine époxy SU-8 3000 commercialisée par la société Micro-Chem.

Une fois la résine réticulée à l'étape ii, on peut de préférence éliminer, à l'aide d'un solvant approprié, la partie de la résine qui n'a pas été irradiée.

L'angle choisi pour l'irradiation à l'étape ii définit l'angle d'inclinaison des projections par rapport à la normale au plan constitué par ladite surface texturée (figure 4 (E)). La lumière étant réfractée dans le substrat transparent utilisé, l'angle des piliers n'est pas toujours exactement l'angle de la lumière ; cela dépend de la nature du matériau constituant le substrat. L'homme du métier ajustera donc l'angle incident en fonction du substrat utilisé de manière à obtenir l'inclinaison des piliers souhaitée.

L'angle choisi pour l'irradiation à l'étape ii peut notamment être inférieur à 90°, de préférence inférieur à 45°, et plus préférentiellement compris entre 10° et 45°.

Le matériau antiadhésif optionnellement ajouté à l'étape iii peut, par exemple, être du trimethylchlorosilane (TMCS).

Enfin, sur la base des moules en PDMS ainsi obtenus, la surface tridimensionnelle anisotrope selon l'invention est réalisée par moulage d'un matériau (PDMS ou PLGA) constituant ladite structure tridimensionnelle selon l'invention, par exemple par coulage d'un polymère siliconé sur le moule en silicone préalablement mis sous vide, ou par moulage thermique dans le cas d'un copolymère poly(acide lactic-co- glycolique) (PLGA).

Après durcissement du matériau constituant la surface tridimensionnelle, le moule en PDMS est séparé de l'échantillon de surface tridimensionnelle présentant ainsi un réseau de projections inclinées par rapport à la normale au plan constitué par ladite surface texturée,

dans la direction conférée par ladite structure anisotrope (Figure 4 (J)).

Lorsque la surface tridimensionnelle anisotrope selon l'invention est réalisée par moulage thermique d'un copolymère PLGA, ledit moulage est opéré à une température comprise entre 80 et 100°C (de préférence de 60 à 90°, et plus préférentiellement environ 90°C), et à une pression comprise entre 60 et 120 bars (de préférence entre 100 et 120 bars, et plus préférentiellement environ 120 bars), pendant environ 10 minutes (Figure 4 (I)). Après refroidissement et abaissement de la pression jusqu'à atteindre la pression atmosphérique, le moule en PDMS est séparé de l'échantillon de surface tridimensionnelle.

La hauteur, le diamètre, l'angle d'inclinaison des projections peuvent ainsi être modulés par l'homme du métier en ajustant différents paramètres du procédé.

Surface support

Dans le cadre du dispositif selon l'invention, les cellules dont la migration est contrôlée sont véhiculées sur un substrat appelé « surface support ».

La surface support sur laquelle reposent les cellules peut être la surface texturée précédemment décrite, ou une surface artificielle telle qu'une surface de culture cellulaire (par exemple un gel), une lamelle de verre, l'intérieur d'un canal microfluidique, ou une surface de l'environnement naturel desdites cellules telle que la surface d'un tissu vivant ou la surface d'une plaie.

Selon un mode particulier de réalisation de l'invention, les cellules peuvent être guidées en les confinant entre leur surface support et ladite surface texturée.

En effet, le confinement des cellules permet de renforcer le guidage des cellules, en particulier lorsque le substrat sur lequel les cellules sont véhiculées est non adhésif.

Dans ce mode de réalisation, la distance entre ladite surface support et ladite surface texturée est comprise entre 0 et 10 $\mu$m, de préférence entre 3 et 6 $\mu$m de sorte que l'épaisseur de la cellule après confinement soit au moins comprise entre 3 et 6 $\mu$m pour permettre sa migration.

Lorsque le support sur lequel sont véhiculées les cellules est « mou », c'est-à-dire présente une rigidité inférieure à environ 20 kPa, notamment comprise entre 100 Pa et 20kPa, de préférence entre 500 Pa et 10 kPa, il n'est pas nécessaire qu'il possède des saillies supplémentaires dans la mesure où la surface est suffisamment « molle » pour permettre aux cellules de ne pas être écrasées par le substrat nano texturé, les cellules définissant leur espace de confinement en déformant la surface support.

Ces supports « mous » sont de type gel de faible rigidité ou tapis cellulaire. Les gels utilisés peuvent être des gels d'origine artificielle tels que le polyacrylamide (PAM) ou le polyéthylène-glycol-diacrylate (PEGDA) ou encore des gels d'origine naturelle comme le collagène, le matrigel ou l'acide hyaluronique (HA). La rigidité de ces gels peut être ajustée par leur composition et par les conditions de leur réticulation.

A l'inverse, lorsque le support sur lequel sont véhiculées les cellules présente une rigidité supérieure à environ 20 kPa, il est souhaitable, dans le cadre de la présente invention, que ledit support possède des saillies supplémentaires pour ne pas altérer les cellules.

La surface support et/ou ladite surface texturée peut comprendre une ou plusieurs saillies supplémentaires permettant de contrôler la distance entre les deux dites surfaces.

La hauteur de ces saillies est mesurée par rapport à la surface sur laquelle elles sont disposées.

Les saillies supplémentaires peuvent notamment se présenter sous la forme de piliers de diamètre compris entre 100 et 500 $\mu$m, et de hauteur comprise entre 1 et 10 $\mu$m, de préférence entre 3 et 6 $\mu$m, et en tout état de cause, de hauteur telle que l'épaisseur de la cellule après confinement est au moins comprise entre 3 et 6 $\mu$m lorsque le support présente une rigidité supérieure à environ 20 kPa.

Méthode de guidage de la migration des cellules

L'invention a également pour objet une méthode de guidage de la migration des cellules comprenant la mise en contact des cellules avec un substrat présentant une surface texturée ayant une structure tridimensionnelle anisotrope, ladite structure étant constituée de projections inclinées par rapport à la normale au plan constitué par ladite surface texturée, dans la direction conférée par ladite structure anisotrope.

Dans cette méthode, la surface texturée est telle que décrite précédemment.

Les cellules sont également véhiculées sur une surface support telle que décrite précédemment de sorte que les cellules peuvent être confinées entre ladite surface texturée et ladite surface support.

L'invention a encore pour objet un dispositif comprenant un substrat présentant une surface texturée destinée à être mise en contact avec des cellules, ladite surface texturée ayant une structure tridimensionnelle anisotrope constituée d'un réseau de projections inclinées par rapport à la normale au plan constitué par ladite surface texturée, dans la direction conférée par ladite structure anisotrope, pour son utilisation dans une méthode de guidage de la migration cellulaire.

Applications

Le dispositif selon l'invention peut notamment trouver de nombreuses applications de guidage de la migration des cellules in vivo ou in vitro.

On entend par guidage in vitro le guidage de la migration des cellules en culture dans un milieu entièrement artificiel.

Les cellules peuvent par exemple être cultivées sur une surface support artificielle telle qu'une surface de culture cellulaire (par exemple un gel), la surface texturée étant alors appliquée sur la surface support pour confiner les cellules.

Dans un autre mode de réalisation, la surface texturée peut être intégrée sur l'une des faces d'un canal microfluidique pour guider la migration des cellules dans ledit canal microfluidique.

Les surfaces texturées du dispositif selon l'invention peuvent être utilisées, soit pour l'étude des mécanismes biologiques et physiques de migration et de prolifération des cellules en culture, soit pour réaliser du tri cellulaire en séparant les cellules suivant leurs caractéristiques de migration.

Alternativement, le dispositif selon l'invention peut permettre de guider des cellules sur des supports bidimensionnels ou tridimensionnels couvert au moins partiellement d'une surface de guidage texturée pour la réalisation d'organes artificiels (ingénierie tissulaire).

Le dispositif selon l'invention peut trouver une application dans tout domaine nécessitant de guider les cellules artificiellement et indépendamment de leur comportement chimiotactique.

On entend par guidage in vivo le guidage de la prolifération et de la migration des cellules chez un être vivant, par exemple chez l'homme.

Dans ce cas, la surface support est constituée du support physiologique naturel des cellules sur lequel est appliquée la surface texturée.

Selon un mode préféré de réalisation de guidage in vivo, la surface texturée peut être utilisée pour guider les cellules présentes à la surface d'une plaie de manière à favoriser la répartition des cellules sur la plaie. Le dispositif est alors un pansement présentant des microstructures ou des nanostructures à sa surface.

Selon un autre mode de réalisation de guidage in vivo, la surface texturée peut être utilisé pour guider les cellules autour d'une prothèse de manière à favoriser la répartition des cellules autour de la prothèse.

Selon encore un autre mode de réalisation de guidage in vivo, la surface texturée peut être utilisé pour guider les cellules autour d'un film ou d'un pansement interne placé à l'intérieur du corps d'un être vivant de manière à favoriser la répartition des cellules dans ou autour d'un organe.

Le dispositif selon l'invention peut notamment se présenter sous la forme d'un pansement, d'un implant, d'une prothèse, d'un support de tissus artificiels, d'un canal microfluidique, d'un laboratoire sur puce intégrant des canaux, et de préférence, ledit dispositif est un pansement.

Exemple

L'effet de guidage de la migration cellulaire du dispositif selon l'invention a été établi sur des cultures cellulaires de fibroblastes normaux de peau humaines (NHDF) en laboratoire.

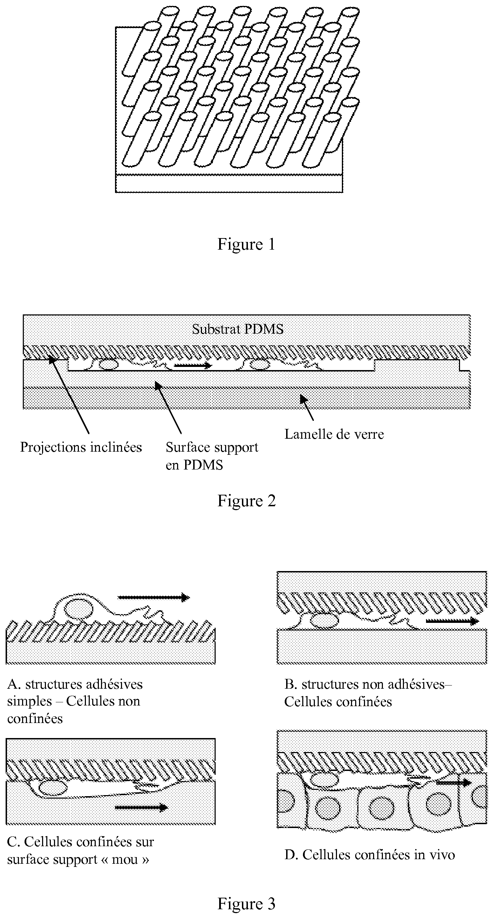

Des cultures cellulaires de NHDF ont été couvertes avec une surface en silicone (polydiméthylsiloxane PDMS) tapissée d'un réseau carré de cylindres inclinés. Le réseau de cylindres inclinés présente les propriétés suivante : 4 $\mu$m de période, 1,5 $\mu$m de diamètre, 5 $\mu$m de hauteur et présentant une inclinaison de 40° par rapport à la normale au plan constitué par ladite surface (voir figure 2).

Le réseau de cylindres inclinés a été préparé par coulage d'un polymère de silicone PDMS sur un moule en silicone (PDMS), comme illustré à la figure 4.

Les moules en silicone (PDMS) ont été préparés à partir d'un moule réalisé par lithographie optique auto-alignée.

Pour cela, un masque optique constitué d'une plaque de verre recouverte d'une couche fine de chrome et d'une couche de résine AZ1518, commercialisé par MicroChem, référencé par PR-AZ1518 Cr) est utilisé comme substrat de base.

Des motifs, en l'espèce une matrice de trous ont été réalisés sur ladite couche de résine photosensible par balayage d'un faisceau laser focalisé sur ladite couche photosensible (figure 4 (B)) suivie d'une étape de développement de la résine.

Puis, on transfère ces motifs sur la couche de chrome, absorbant la lumière par gravure humide (ChromeEtch) et on élimine les motifs de résine avec de l'isopropanol (figure 4 (C))

On a ensuite déposé, sur la plaque de verre comprenant les motifs précédemment définis, une couche de résine épaisse époxy SU-8 3005 commercialisée par la société MicrChem, d'épaisseur de l'ordre de 5$\mu$m (figure 4 (D)).

La plaque de verre comprenant ladite couche de résine a ensuite été retournée pour être irradiée par UV du côté de la plaque de verre, avec un angle incident d'environ 70° de manière à ce que l'angle d'inclinaison des cylindres par rapport à la normale au plan constitué par ladite surface texturée soit de 40° (figure 4 (E)).

Après révélation des cylindres inclinés à la surface de la résine, ladite surface tridimensionnelle de résine ainsi formée a été recouverte d'un matériau antiadhésif (trimethylchlorosilane ou TMCS).

La surface tridimensionnelle ainsi formée a alors été utilisée pour réaliser un « contre-moule » en silicone (PDMS) de manière à créer des copies des moules en négative. Pour cela, une couche de d'un mélange de PDMS/réticulant (RTV615A, General electric, réticulation à 70° pendant 1 h) est coulée sur la surface de résine traitée antiadhésive (Figure 4 (G)). Une fois réticulé, la couche de PDMS est décollée de la surface tridimensionnelle, formant ainsi un moule en négatif de ladite surface (Figure 4 (H)).

Enfin, le réseau de cylindres inclinés selon l'invention est obtenu par coulage d'un mélange de PDMS/réticulant (RTV615A, General electric, réticulation à 70° pendant 1 h) sur le moule après avoir placé le moule sous vide pendant 1h (figure 5).

La surface en silicone est, par ailleurs, soutenue par de larges piliers de 5 μm de hauteur et de 400 μm de diamètre positionnés tous les millimètres et moulés dans du PDMS.

Les cellules en culture se trouvent donc en contact avec les cylindres inclinés, sans être totalement écrasées par ceux-ci, ayant un espace de 5 μm entre les 2 surfaces pour migrer de manière confinée (figure 2).

La surface en silicone tapissée du réseau de cylindres inclinés est rendue non adhésive par un traitement chimique de greffage d'un copolymère de poly(L-lysine) et de poly éthylène glycol (PLL-g-PEG).

Le substrat de culture des cellules (NHDF) est, quant à lui, également constitué de PDMS traité avec de la fibronectine (50 μg/ml).

De manière surprenante, il a été observé que les NHDF migraient sous la surface tapissée de cylindres inclinés préférentiellement dans la direction d'inclinaison des cylindres. La figure 6 représente un histogramme de la direction moyenne de migration des cellules pendant 24h. Chaque élément de l'ensemble représenté indique la direction moyenne d'une cellule sur un laps de temps de 24h après le confinement. 75 cellules sont ici représentées

Cet effet à également été observé pour différents réseaux de projections ayant un diamètre de 1,5 μm, les hauteurs *h,* distances *d* entre les projections, et angles d'inclinaison $\alpha$ par rapport à la normale suivants :

$$(h, d, a) = (7 \text{ μm, } 4 \text{ μm, } 80°),$$

$$(h, d, a) = (7,5 \text{ μm, } 7 \text{ μm, } 60°),$$

$$(h, d, a) = (5 \text{ μm, } 5 \text{ μm, } 45°),$$

$$(h, d, a) = (7 \text{ μm, } 4 \text{ μm, } 80°),$$

$$(h, d, a) = (4 \text{ μm, } 4 \text{ μm, } 60°),$$

$$(h, d, a) = (4 \text{ μm, } 6,5 \text{ μm, } 60°),$$

$$(h, d, a) = (4 \text{ μm, } 9 \text{ μm, } 60°)$$

et

$$(h, d, a) = (4 \text{ μm, } 11,5 \text{ μm, } 60°)$$

les projections étant réalisées dans différents matériaux (PDMS, acide poly(lactique-co-glycolique) PLGA).

**Revendications**

1. Dispositif de guidage de la migration cellulaire comprenant un substrat présentant une surface texturée destinée à être mise en contact avec des cellules, ladite surface texturée ayant une structure tridimensionnelle anisotrope constituée d'un réseau de projections inclinées par rapport à la normale au plan constitué par ladite surface texturée, dans la direction conférée par ladite structure anisotrope.

2. Dispositif de guidage selon la revendication 1, **caractérisé en ce que** ledit réseau de projections présente une période inférieure à la taille des cellules, de préférence inférieure à 20 μm, de préférence encore comprise entre 0,1 et 15 μm, plus préférentiellement entre 5 et 10 μm et encore plus préférentiellement de l'ordre de 5 μm.

3. Dispositif de guidage selon l'une quelconque des revendications 1 ou 2, **caractérisé en ce que** les projections inclinées présentent un angle d'inclinaison inférieur ou égal à 45° par rapport à la normale au plan constitué par ladite surface texturée, de préférence entre 10° et 45°.

4. Dispositif de guidage selon l'une quelconque des revendications 1 à 3, **caractérisé en ce que** les projections inclinées présentent un rapport d'aspect, correspondant au rapport de la hauteur sur le diamètre des projections, compris entre 0,5 et 20, de préférence entre 2 et 10.

5. Dispositif de guidage selon l'une quelconque des revendications 1 à 4, **caractérisé en ce que** les projections inclinées se présentent sous la forme de cylindre présentant notamment une section sphérique ou ovoïdale, de cônes, de pyramides, de lamelles présentant notamment une section rectangulaire ou de la forme d'un parallélogramme, ou d'écailles de forme sensiblement triangulaire, semi-elliptique, semi-circulaire.

6. Dispositif de guidage selon l'une quelconque des revendications 1 à 5, **caractérisé en ce que** les projections inclinées se présentent sous la forme de cylindres de diamètre compris entre 10 nm et 10 μm, de préférence entre 0,5 et 3 μm.

7. Dispositif de guidage selon l'une quelconque des revendications 1 à 6, **caractérisé en ce que** le substrat présentant une surface texturée est adhésif.

8. Dispositif de guidage selon l'une quelconque des revendications 1 à 6, **caractérisé en ce que** le substrat présentant une surface texturée est non adhésif.

9. Dispositif de guidage selon la revendication 8, **ca-**

**ractérisé en ce que** le substrat non adhésif est constitué d'un matériau non adhésif tel qu'un polymère fluoré ou d'un matériau rendu non adhésif par traitement chimique tel que le greffage de molécules de polyéthylène glycol (PEG).

10. Dispositif de guidage selon l'une quelconque des revendications 1 à 9, **caractérisé en ce qu'**il se présente sous la forme d'un pansement, d'un implant, d'une prothèse, d'un support de tissus artificiels, d'un canal microfluidique, d'un laboratoire sur puce intégrant des canaux, et de préférence, ledit dispositif est un pansement.

11. Méthode de guidage in vitro de la migration des cellules comprenant la mise en contact des cellules avec un substrat présentant une surface texturée ayant une structure tridimensionnelle anisotrope, ladite structure étant constituée d'un réseau de projections inclinées telles que définies dans les revendications 1 à 9.

12. Méthode de guidage selon la revendication 11, **caractérisée en ce que** les cellules sont véhiculées sur une surface support.

13. Méthode de guidage selon la revendication 12, **caractérisée en ce que** la surface support est une surface artificielle telle qu'une surface de culture cellulaire (par exemple un gel), une lamelle de verre, l'intérieur d'un canal microfluidique, ou une surface de l'environnement naturel desdites cellules telle que la surface d'un tissu vivant ou la surface d'une plaie.

14. Méthode de guidage selon l'une quelconque des revendications 12 ou 13, **caractérisée en ce que** les cellules sont confinées entre ladite surface support et ladite surface texturée.

15. Méthode selon l'une quelconque des revendications 12 à 14, **caractérisée en ce que** la distance entre ladite surface support et ladite surface texturée est comprise entre 0 et 10 $\mu$m, de préférence entre 3 et 6 $\mu$m.

16. Méthode de guidage selon l'une quelconque des revendications 12 à 15, **caractérisée en ce que** ladite surface support et/ou ladite surface texturée comprend une ou plusieurs saillies supplémentaires permettant de contrôler la distance entre les deux dites surfaces.

17. Méthode de guidage selon la revendication 16, **caractérisée en ce que** les saillies supplémentaires se présentent sous la forme de piliers de diamètre compris entre 100 et 500 $\mu$m, et de hauteur inférieure à 10 $\mu$m, de préférence entre 3 et 6 $\mu$m.

18. Procédé de préparation d'une surface texturée ayant une structure tridimensionnelle anisotrope constituée d'un réseau de projections inclinées selon l'une quelconque des revendications 1 à 6, comprenant :

    i. la préparation de moules en silicone constituant des copies en négatif de ladite structure tridimensionnelle, notamment par lithographie optique auto-alignée,
    ii. le moulage du matériau constituant ladite structure tridimensionnelle,
    iii. le décollement du moule en silicone.

19. Procédé de préparation selon la revendication précédente, **caractérisé en ce que** l'étape i de préparation des moules en silicone (PDMS) est réalisée par lithographie optique auto-alignée, comprenant notamment les étapes suivantes :

    i. le dépôt, d'une couche de résine photosensible sur un masque optique,
    ii. l'irradiation de ladite couche de résine à travers le masque optique par une source lumineuse, avec un angle incident (correspondant à l'angle formé par le rayon incident et la normale au plan constitué par le masque), ledit angle incident conditionnant l'angle d'inclinaison des piliers de la structure tridimensionnelle anisotrope,
    iii. optionnellement, le recouvrement de ladite surface tridimensionnelle de résine par un matériau antiadhésif,
    iv. le coulage d'une couche de PDMS sur ladite surface tridimensionnelle de résine de manière à obtenir lesdits moules en silicone (PDMS),
    v. après polymérisation, décollement du moule de PDMS ainsi obtenu

20. Procédé de préparation selon la revendication précédente, **caractérisé en ce que**, après réticulation de la résine à l'étape ii, on élimine, à l'aide d'un solvant approprié, la partie de la résine qui n'a pas été irradiée.

21. Procédé de préparation selon l'une quelconque des revendications 19 ou 20, **caractérisé en ce que** le matériau antiadhésif optionnellement ajouté à l'étape iii est le triméthylchlorosilane (TMCS).

22. Procédé de préparation selon l'une quelconque des revendications 18 à 21, **caractérisé en ce que** la surface tridimensionnelle anisotrope est réalisée par moulage d'un matériau (PDMS ou PLGA) constituant ladite structure tridimensionnelle selon l'invention, par exemple par coulage d'un polymère siliconé sur le moule en silicone préalablement mis sous vide, ou par moulage thermique dans le cas d'un copolymère poly(acide lactic-co- glycolique) (PLGA).

**23.** Procédé de préparation selon la revendication précédente, **caractérisé en ce que** la surface tridimensionnelle anisotrope est réalisée par moulage thermique d'un copolymère PLGA, opéré à une température comprise entre 80 et 100°C (de préférence de 60 à 90°, et plus préférentiellement environ 90°C), et à une pression comprise entre 60 et 120 bars (de préférence entre 100 et 120 bars, et plus préférentiellement environ 120 bars), pendant environ 10 minutes.

**24.** Procédé de préparation selon la revendication précédente, **caractérisé en ce que** le moule en PDMS est séparé de l'échantillon de surface tridimensionnelle après refroidissement et abaissement de la pression jusqu'à atteindre la pression atmosphérique, ladite surface tridimensionnelle présentant ainsi un réseau de projections inclinées par rapport à la normale au plan constitué par ladite surface texturée, dans la direction conférée par ladite structure anisotrope.

**Patentansprüche**

**1.** Vorrichtung zum Führen der Zellmigration, umfassend ein Substrat, welches eine texturierte Oberfläche aufweist, welche dazu vorgesehen ist, in Kontakt mit Zellen gebracht zu werden, wobei die texturierte Oberfläche eine anisotrope dreidimensionale Struktur aufweist, welche durch ein Netz von Vorsprüngen gebildet ist, welche gegenüber der Normalen zu der Ebene geneigt sind, welche durch die texturierte Oberfläche gebildet ist, in die Richtung, welche durch die anisotrope Struktur bestimmt wird.

**2.** Vorrichtung zum Führen nach Anspruch 1, **dadurch gekennzeichnet, dass** das Netz von Vorsprüngen eine Periode aufweist, welche kleiner als die Größe der Zellen ist, vorzugsweise kleiner als 20 $\mu$m, weiter vorzugsweise zwischen 0,1 und 15 $\mu$m beträgt, noch weiter bevorzugt zwischen 5 und 10 $\mu$m und noch weiter bevorzugt in der Größenordnung von 5 $\mu$m liegt.

**3.** Vorrichtung zum Führen nach einem der Ansprüche 1 oder 2, **dadurch gekennzeichnet, dass** die geneigten Vorsprünge einen Neigungswinkel kleiner oder gleich 45° gegenüber der Normalen zu der Ebene aufweisen, welche durch die texturierte Oberfläche gebildet ist, vorzugsweise zwischen 10° und 45°.

**4.** Vorrichtung zum Führen nach einem der Ansprüche 1 bis 3, **dadurch gekennzeichnet, dass** die geneigten Vorsprünge ein Aspektverhältnis aufweisen, entsprechend dem Verhältnis der Höhe zu dem Durchmesser der Vorsprünge, welches zwischen 0,5 und 20 beträgt, vorzugsweise zwischen 2 und 10.

**5.** Vorrichtung zum Führen nach einem der Ansprüche 1 bis 4, **dadurch gekennzeichnet, dass** die geneigten Vorsprünge in Form von Zylindern, insbesondere mit einem sphärischen oder eiförmigem Querschnitt, von Kegeln, von Pyramiden, von Lamellen, insbesondere mit einem rechteckigen oder Parallelogramm-förmigen Querschnitt, oder von Schuppen mit im Wesentlichen dreieckiger, halbelliptischer, halbkreisförmiger Form vorliegen.

**6.** Vorrichtung zum Führen nach einem der Ansprüche 1 bis 5, **dadurch gekennzeichnet, dass** die geneigten Vorsprünge in der Form von Zylindern mit einem Durchmesser vorliegen, welcher zwischen 10 nm und 10 $\mu$m beträgt, vorzugsweise zwischen 0,5 und 3 $\mu$m.

**7.** Vorrichtung zum Führen nach einem der Ansprüche 1 bis 6, **dadurch gekennzeichnet, dass** das Substrat eine texturierte und haftfähige Oberfläche aufweist.

**8.** Vorrichtung zum Führen nach einem der Ansprüche 1 bis 6, **dadurch gekennzeichnet, dass** das Substrat eine texturierte und nicht-haftfähige Oberfläche aufweist.

**9.** Vorrichtung zum Führen nach Anspruch 8, **dadurch gekennzeichnet, dass** das nicht-haftfähige Substrat aus einem nicht-haftfähigen Material gebildet ist, beispielsweise aus einem fluorierten Polymer oder einem Material, welches durch eine chemische Behandlung nicht-haftfähig gemacht ist, beispielsweise ein Aufbringen von Polyethylenglykol (PEG)-Molekülen.

**10.** Vorrichtung zum Führen nach einem der Ansprüche 1 bis 9, **dadurch gekennzeichnet, dass** sie in Form eines Verbands, eines Implantats, einer Prothese, einem Träger für künstliche Gewebe, einem Mikrofluid-Kanal, einem Labor auf einem Chip mit integrierten Kanälen vorliegt, wobei vorzugsweise die Vorrichtung ein Verband ist.

**11.** Verfahren zum in-vitro-Führen der Migration von Zellen, umfassend das in-Kontakt-Bringen der Zellen mit einem Substrat, welches eine texturierte Oberfläche mit einer anisotropen dreidimensionalen Struktur aufweist, wobei die Struktur aus einem Netz von geneigten Vorsprüngen gebildet ist, wie in den Ansprüchen 1 bis 9 definiert.

**12.** Verfahren zum Führen nach Anspruch 11, **dadurch gekennzeichnet, dass** die Zellen auf einer Trägerfläche transportiert werden.

**13.** Verfahren zum Führen nach Anspruch 12, **dadurch gekennzeichnet, dass** die Trägerfläche eine künstliche Oberfläche ist, wie beispielsweise eine Zellkultur-Oberfläche (beispielsweise ein Gel), eine Glaslamelle, der Innenraum eines Mikrofluid-Kanals oder eine Oberfläche der natürlichen Umgebung der Zellen, wie beispielsweise eine Oberfläche eines lebenden Gewebes oder die Oberfläche einer Wunde.

14. Verfahren zum Führen nach einem der Ansprüche 12 oder 13, **dadurch gekennzeichnet**, das die Zellen zwischen der Tragefläche und der texturierten Oberfläche eingeschlossen werden.

15. Verfahren nach einem der Ansprüche 12 bis 14, **dadurch gekennzeichnet, dass** der Abstand zwischen der Trägerfläche und der texturierten Oberfläche zwischen 0 und 10 $\mu$m beträgt, vorzugsweise zwischen 3 und 6 $\mu$m.

16. Verfahren zum Führen nach einem der Ansprüche 12 bis 15, **dadurch gekennzeichnet, dass** die Trägerfläche und/oder die texturierte Oberfläche einen oder mehrere zusätzliche Ausbuchtungen umfasst, was ein Einstellen des Abstands zwischen den beiden Oberflächen erlaubt.

17. Verfahren zum Führen nach Anspruch 16, **dadurch gekennzeichnet, dass** die zusätzlichen Ausbuchtungen in Form von Säulen vorliegen, deren Durchmesser zwischen 100 und 500 $\mu$m beträgt, und deren Höhe kleiner als 10 $\mu$m ist, vorzugsweise zwischen 3 und 6 $\mu$m beträgt.

18. Verfahren zum Herstellen einer texturierten Oberfläche mit einer anisotropen dreidimensionalen Struktur, welche durch ein Netz von geneigten Vorsprüngen gebildet ist, nach einem der Ansprüche 1 bis 6, umfassend:

   i. das Herstellen von Formen aus Silikon, welche Negativkopien der dreidimensionalen Struktur bilden, insbesondere durch selbstausgerichtete optische Lithographie,
   ii. das Gießen des Materials, welches die dreidimensionale Struktur bildet,
   iii. das Entfernen der Form aus Silikon.

19. Verfahren zum Herstellen nach dem vorhergehenden Anspruch, **dadurch gekennzeichnet, dass** der Schritt i des Herstellens von Formen aus Silikon (PDMS) mittels selbstausgerichteter optischer Lithographie durchgeführt wird, insbesondere umfassend die folgenden Schritte:

   i. das Anlagern einer Schicht von fotosensitivem Harz an einer optischen Maske,
   ii. das Belichten der Harzschicht durch die optische Maske durch eine Lichtquelle mit einem Einfallswinkel (entsprechend dem Winkel, welcher durch den einfallenden Strahl und die Normale auf die Ebene, die durch die Maske gebildet ist, ausgebildet ist), wobei der Einfallswinkel den Neigungswinkel der Säulen der anisotropen dreidimensionalen Struktur bewirkt,
   iii. optional das Abdecken der dreidimensionalen Harzoberfläche durch ein Antihaft-Material,
   iv. Aufbringen einer Schicht aus PDMS auf der dreidimensionalen Harzoberfläche derart, dass die Formen aus Silikon (PDMS) erhalten werden,
   v. nach dem Polymerisieren Ablösen der so erhaltenen Form aus PDMS.

20. Verfahren zum Herstellen nach dem vorhergehenden Anspruch, **dadurch gekennzeichnet**, das nach dem Vernetzen des Harzes in Schritt ii unter Zuhilfenahme eines geeigneten Lösungsmittels der Teil des Harzes beseitigt wird, der nicht belichtet worden ist.

21. Verfahren zum Herstellen nach einem der Ansprüche 19 oder 20, **dadurch gekennzeichnet, dass** das in Schritt iii optional zugefügte Antihaft-Material Trimethylchlorsilan (TMCS) ist.

22. Verfahren zum Herstellen nach einem der Ansprüche 18 bis 21, **dadurch gekennzeichnet, dass** die anisotrope dreidimensionale Oberfläche durch Gießen eines Materials (PDMS oder PLGA) durchgeführt wird, welches die erfindungsgemäße dreidimensionale Struktur bildet, beispielsweise durch Aufbringen eines Silikonpolymers auf die zuvor evakuierte Form aus Silikon, oder durch thermisches Gießen in dem Fall eines Poly(lactid-co-glycolid)-Copolymers (PLGA).

23. Verfahren zum Herstellen nach dem vorhergehenden Anspruch, **dadurch gekennzeichnet, dass** die anisotrope dreidimensionale Oberfläche durch thermisches Gießen eines PLGA-Copolymers hergestellt wird, betrieben bei einer Temperatur, welche zwischen 80 und 100°C beträgt (vorzugsweise von 60 bis 90° und weiter bevorzugt etwa 90°C), sowie einem Druck, welcher zwischen 60 und 120 bar beträgt (vorzugsweise zwischen 100 und 120 bar und weiter bevorzugt etwa 120 bar), während etwa 10 Minuten.

24. Verfahren zum Herstellen nach dem vorhergehenden Anspruch, **dadurch gekennzeichnet, dass** die Form aus PDMS von dem Muster der dreidimensionalen Oberfläche nach einem Abkühlen und Verringern des Drucks bis zum Erhalten des Atmosphärendrucks abgetrennt wird, wobei die dreidimensionale Oberfläche somit ein Netz von Vorsprüngen umfasst, welche bezüglich der Normalen zu der Ebene geneigt sind, welche durch die texturierte Oberfläche gebildet ist, in die Richtung, welche durch die anisotrope Struktur festgelegt ist.

## Claims

1. A device for guiding cell migration, comprising a substrate having a textured surface intended to be brought into contact with cells, said textured surface having an anisotropic three-dimensional structure consisting of a network of projections inclined relative to the normal to the plane formed by said textured surface, in the direction imparted by said anisotropic structure.

2. The guiding device as claimed in claim 1, **characterized in that** said network of projections has a spacing which is smaller than the size of the cells, preferably less than 20 μm, more preferably between 0.1 and 15 μm, more preferentially between 5 and 10 μm and even more preferentially about 5 μm.

3. The guiding device as claimed in either one of claims 1 and 2, **characterized in that** the inclined projections have an angle of inclination of less than or equal to 45° relative to the normal to the plane formed by said textured surface, preferably between 10° and 45°.

4. The guiding device as claimed in any one of claims 1 to 3, **characterized in that** the inclined projections have an aspect ratio, corresponding to the ratio of the height to the diameter of the projections, of between 0.5 and 20, preferably between 2 and 10.

5. The guiding device as claimed in any one of claims 1 to 4, **characterized in that** the inclined projections are in the shape of a cylinder having in particular a spherical or ovoid cross section, of cones, of pyramids, of lamellae having in particular a cross section which is rectangular or in the shape of a parallelogram, or of flakes of substantially triangular, semielliptical or semicircular shape.

6. The guiding device as claimed in any one of claims 1 to 5, **characterized in that** the inclined projections are in the shape of cylinders with a diameter of between 10 nm and 10 μm, preferably between 0.5 and 3 μm.

7. The guiding device as claimed in any one of claims 1 to 6, **characterized in that** the substrate having a textured surface is adhesive.

8. The guiding device as claimed in any one of claims 1 to 6, **characterized in that** the substrate having a textured surface is non-adhesive.

9. The guiding device as claimed in claim 8, **characterized in that** the non-adhesive substrate consists of a non-adhesive material such as a fluoropolymer or of a material made non-adhesive by chemical treatment, such as the grafting of molecules of polyethylene glycol (PEG).

10. The guiding device as claimed in any one of claims 1 to 9, **characterized in that** it is in the form of a dressing, an implant, a prosthesis, an artificial-tissue support, a microfluidic channel, or a Lab-on-a-chip device integrating channels, and preferably said device is a dressing.

11. A method for in vitro guiding cell migration, comprising bringing the cells into contact with a substrate having a textured surface which has an anisotropic three-dimensional structure, said structure consisting of a network of inclined projections as defined in claims 1 to 9.

12. The guiding method as claimed in claim 11, **characterized in that** the cells are conveyed on a support surface.

13. The guiding method as claimed in claim 12, **characterized in that** the support surface is an artificial surface such as a cell culture surface (for example a gel), a glass coverslip, the inside of a microfluidic channel, or a surface of the natural environment of said cells, such as the surface of a living tissue or the surface of a wound.

14. The guiding method as claimed in either one of claims 12 and 13, **characterized in that** the cells are confined between said support surface and said textured surface.

15. The method as claimed in any one of claims 12 to 14, **characterized in that** the distance between said support surface and said textured surface is between 0 and 10 μm, preferably between 3 and 6 μm.

16. The guiding method as claimed in any one of claims 12 to 15, **characterized in that** said support surface and/or said textured surface comprises one or more additional protuberances which make it possible to control the distance between the two said surfaces.

17. The guiding method as claimed in claim 16, **characterized in that** the additional protuberances are in the form of pillars with a diameter of between 100 and 500 μm, and a height of less than 10 μm, preferably between 3 and 6 μm.

18. A process for preparing a textured surface which has an anisotropic three-dimensional structure consisting of a network of inclined projections as claimed in any one of claims 1 to 6, comprising:

> i. the preparation of silicone molds constituting negative copies of said three-dimensional structure, in particular by self-aligned optical lithography,
> ii. the molding of the material constituting said three-dimensional structure,
> iii. the detachment of the silicone mold.

19. The preparation process as claimed in the preceding claim, **characterized in that** step i of preparation of the silicone (PDMS) molds is carried out by self-aligned optical lithography, comprising in particular the following steps:

> i. the depositing of a layer of photosensitive resin on an optical mask,
> ii. the irradiation of said layer of resin through the optical mask using a light source, with an incident angle (corresponding to the angle formed by the incident ray and the normal to the plane formed by the mask), said incident angle conditioning the angle of inclination of the pillars of the anisotropic three-dimensional structure,
> iii. optionally, the covering of said three-dimensional surface of resin with an anti-fouling material,
> iv. the casting of a layer of PDMS on said three-dimensional surface of resin so as to obtain said silicone (PDMS) molds,
> v. after polymerization, detachment of the resulting PDMS mold.

20. The preparation process as claimed in the preceding claim, **characterized in that**, after crosslinking of the resin in step ii, the part of the resin which has not been irradiated is removed using an appropriate solvent.

21. The preparation process as claimed in either one of claims 19 and 20, **characterized in that** the antifouling material optionally added in step iii is trimethylchlorosilane (TMCS).

22. The preparation process as claimed in any one of claims 18 to 21, **characterized in that** the anisotropic three-dimensional surface is produced by molding of a material (PDMS or PLGA) constituting said three-dimensional structure as claimed in the invention, for example by casting a silicone polymer on the silicone mold placed under vacuum beforehand, or by thermal molding in the case of a poly(lactic-co-glycolic acid) (PLGA) copolymer.

23. The preparation process as claimed in the preceding claim, **characterized in that** the anisotropic three-dimensional surface is produced by thermal molding of a PLGA copolymer, carried out at a temperature of between 80 and 100°C (preferably from 60 to 90°C, and more preferentially approximately 90°C), and at a pressure of between 60 and 120 bar (preferably between 100 and 120 bar, and more preferentially approximately 120 bar), for approximately 10 minutes.

24. The preparation process as claimed in the preceding claim, **characterized in that** the PDMS mold is separated from the sample of three-dimensional surface after cooling and reduction of the pressure to reach atmospheric pressure, said three-dimensional surface thus having a network of projections inclined relative to the normal to the plane formed by said textured surface, in the direction imparted by said anisotropic structure.

Figure 1

Projections inclinées    Surface support
en PDMS

Lamelle de verre

Figure 2

A. structures adhésives
simples – Cellules non
confinées

B. structures non adhésives–
Cellules confinées

C. Cellules confinées sur
surface support « mou »

D. Cellules confinées in vivo

Figure 3

UV

Résine photosensible

Chrome

Figure 4 (A)

Plaque de verre

Figure 4 (B)

Plaque de verre

Figure 4 (C)

Résine épaisse

Plaque de verre

Figure 4 (D)

UV

Plaque de verre

$\partial$

Figure 4 (E)

Piliers formés dans la résine épaisse

Plaque de verre

Figure 4 (F)

PDMS

Plaque de verre

Figure 4 (G)

PDMS

Figure 4 (H)

Matériau
constituant ladite
structure
tridimensionnelle

PDMS

Figure 4 (I)

Figure 4 (J)

Figure 5

Figure 6

18

**EP 2 758 520 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1199354 A
- US 20070009572 A
- US 200902481445 A
- US 20090093879 A

### Littérature non-brevet citée dans la description

- **MAHMUD et al.** *Nature Physics,* 2009, vol. 4, 606